# EUROPEAN PATENT SPECIFICATION

(11) **EP 1 013 242 B1**
(45) Date of publication and mention of the grant of the patent: **19.10.2005**
(21) Application number: 99310199.7
(22) Date of filing: 16.12.1999
(51) Int. Cl.: A61F 2/36, A61F 2/46

(54) **Femoral component**
Femurkomponente
Composant fémoral

(30) Priority: 18.12.1998 GB 9828084
(43) Date of publication of application: 28.06.2000
(73) Proprietor: BENOIST GIRARD SAS, 14200 Hérouville-Saint-Clair (FR)
(72) Inventor: Ling, Robin Sydney Mackwood, Dartmouth, Devon TQ6 0HB (GB); Gie, Graham Allan, Yeoford, Devon EX17 5EZ (GB); Timperley, Andrew John, St. Leonard's, Exeter EX2 4PA (GB); Storer, John Andrew, 14250 Jouaye Mondaye, Bayeux (FR)
(74) Representative: Bridge-Butler, Alan James

(56) References cited:
- EP-A- 0 000 549
- EP-A- 0 485 311
- EP-A- 0 636 351
- EP-A- 0 845 251
- WO-A-97/20525
- FR-A- 2 689 001
- US-A- 5 137 535

## Description

This invention relates to a femoral component of a replacement hip joint of the "Exeter" type which has a collarless stem including a shoulder and for fixing in a medullary canal by cement.

The "Exeter" type femoral component of the kind shown in British Patent No 1 409 054 is well known and comprises a neck which carries a ball head for cooperation with an acetabular socket. The neck is connected to a tapered collarless stem. Thus there is no collar for resting either on the bone or the cement in the area where the stem joins the neck of the implant. This type of stem has evolved so that the stem can be given a polished finish to help it slide down inside the bone cement and the present invention relates to this type of femoral component.

In certain circumstances it can be difficult to locate the femoral component in the medullary socket with the neck and ball at the precise height and angle required. This can be caused by the bone which are structurally imperfect or by difficulties in reaming a suitable cavity. It is particularly pronounced when cavities have to be lined with bone fragments prior to cementing or when there are irregularities and the present invention is intended to overcome some of the difficulties experienced.

It is known from EP-A-0 845 251 to provide a femoral component with a separate proximal component but in this Application the angle of the proximal component with regard to the stem about a proximal/distal axis is fixed and there is no provision for adjustment.

US Patent Specification 4 051 559 also shows the use of a separate proximal component and this is provided to allow it to be placed in position on a cylindrical stem which is intended to be screwed directly into the bone. The proximal component is provided with a collar which is intended to rest against the cut and prepared bone and there is no provision for a stem to slide down inside bone cement as is required by the Exeter type component. Moreover, the angular adjustment about a proximal/distal axis is much too coarse to allow final accurate adjustment with an Exeter type component.

FR-A-2 689 001 shows a femoral component comprising a stem and a proximal component which is attachable to it via a tapered boss and a socket In order to maintain the parts in position an axially extending screw is provided which extends through the proximal component and axially into the boss.

A problem with such constructions is that there is a possibility of the proximal component twisting on the tapered boss as the locating screw is tightened down.

EP-A-0 845 251 shows a rather more complex structure but similar to FR-A-2 689 001 in as much that a proximal component carrying a neck and head is secured on a taper to a stem by means of an axially extending locking bolt and the same problem of relative movement can again arise during locking because the locking screw is concentric with the taper.

According to the present invention a femoral component of a replacement hip joint has a stem for fixing in cement in a medullary cavity by cement and having a separate proximal component provided with a neck for a ball head, and including attachment means for securing the proximal component to the stem which allows selection of an infinite number of angularly displaced positions about a proximal/distal axis, and adapted to allow fixation of the two parts after insertion of the stem into the prepared medullary cavity and without causing torsional loads on the stem characterised in that said attachment means include a boss (20) provided on a proximal portion of said stem (1), a cavity in said proximal component (2) to receive and co-operate with said boss (20) and releasable locking means (25) extending into the cavity and which engage a side of the boss (20) in a direction substantially normal to the longitudinal axis thereof to prevent rotatable axial movement after initial fixing.

Thus, with the component according to the present invention, it is possible to insert the stem into a prepared medullary cavity and subsequently add the proximal component at the precise angle about the proximal/distal axis and then complete fixation of the two parts without causing torsional loads on the stem which might cause the stem to move in the cement and create cavities or cause other damage. This also prevents the stem from being twisted about the proximal/distal axis which might alter the precisely prepared angle of the proximal component

In a convenient construction the boss can be tapered to accept a tapered cavity on the proximal component and the releasable locking means may comprise an axially extending locking screw extending from the proximal component into the tapered boss.

Alternatively the releasable locking means may comprise cotter pin adapted to locate on a boss extension, the pin extending tangentially to the extension.

In another construction the releasable locking means can be provided by a circumferential groove in the boss which can be engaged by a radially extending lock pin or screw in the proximal component.

According to another preferred construction the boss can be cylindrical and with this arrangement the releasable locking means can again be provided by a tangentially extending cotter pin located in the proximal component.

If desired the cotter pin can be located in a groove in the boss.

With these arrangements the releasable locking means may comprise a radially extending pin or screw in the proximal component which can engage a peripheral groove in the boss.

In another alternative construction the boss can be cylindrical or tapered and be provided with a peripheral groove and the releasable locking means can comprise a locking plate with means to move it radially to engage said groove.

The invention can be performed in many ways and some embodiments will now be described by way of example and with reference to the accompanying drawings in which:
Figure 1 is a part cross-sectional diagrammatic side elevation of a femoral component using a collarless stem for fixing in cement in a medullary cavity according to the present invention;
Figure 2 is a side elevation of a cotter pin for use in the construction shown in Figure 1;
Figure 3 is a part cross-sectional side elevation of an alternative construction;
Figure 4 is a part cross-sectional side elevation of another alternative construction using a cylindrical boss;
Figure 5 is a side elevation of a cotter pin for use with the construction shown in Figure 4;
Figure 6 is a part cross-sectional side elevation of yet another alternative construction;
Figure 7 is a side elevation of a cotter pin for use in the construction shown in Figure 6;
Figure 8 is another part cross-sectional side elevation of another alternative construction;
Figure 9 is a part cross-sectional side elevation of yet a further alternative construction; and,
Figure 10 is a plan view of a locking plate for use in the construction shown in Figure 10.

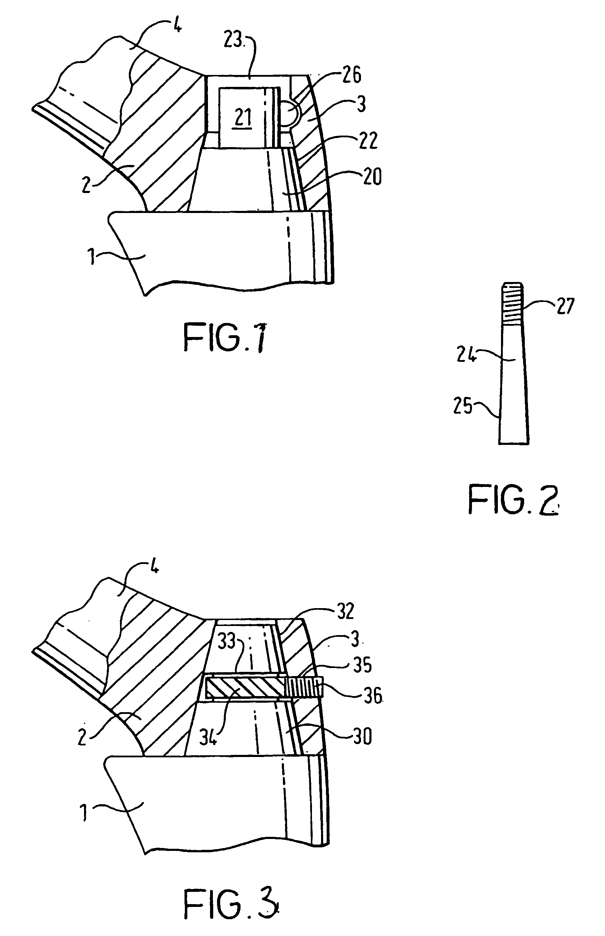

As shown in Figures 1 and 2 of the drawing the femoral component comprises two parts, a shoulderless stem 1 and a proximal component 2. The proximal component has an engagement portion 3 and a neck 4 with a tapered spigot (not shown) to receive a ball head.

The portion 3 is provided with a tapered cavity or socket 22 which is adapted to co-operate with a tapered boss 20 provided on the proximal end of the stem 1. The taper can be a morse taper so that when pressed together the two parts tend to lock in position.

The boss has a cylindrical extension 21 and the tapered socket or cavity 22 extends upwardly as a cylindrical opening 23.

In order to prevent the taper inadvertently coming apart and during use releasable locking means are provided which, in this construction include a cotter pin 24 as shown in Figure 3. This cotter pin has a flat side 25 and is shaped to pass through a hole 26 provided in the portion 3 and shaped so that the flat side of the tapered pin 24 engages the cylindrical extension 21, the flat side being tangential to the extension.

In order to insert the femoral component according to the invention the medullary cavity of the bone to which it is to be fitted is suitably prepared and lined with cement. The stem 1 is now inserted, perhaps using an appropriate inserter, but due to the construction of the present invention its angular position about a proximal/distal axis is not vital and it can therefore be inserted so that it can take up the best and appropriate position in the cavity. Thus, it can be positioned so that it has the maximum amount of cement about it and so that it is not close to any of the cavity walls.

With the stem located in the cavity the proximal component can be carefully fitted so that it projects angularly about the proximal/distal axis as is required. In order to hold the tapers in position it may be necessary to deliver a light blow in the proximal/distal axis direction to ensure that the tapers seat and grip.

In order to locate the proximal component in place the cotter pin is inserted through the opening 26 and tightened by a nut (not shown) on a threaded portion 27. This pulls the cotter pin through on its taper firmly engaging it with the extension 21.

it will be seen that no torsional twisting movement is applied to the stem 1 as the releasable locking means are placed in position.

In Figure 3 the same reference numerals are again used to indicate similar parts to those shown in Figure 1 but in this construction an extended tapered boss 30 is used and the socket 32 extends through the portion 3. The tapered boss 30 has a groove 33 which is lined with a bonded synthetic plastics material indicated by reference numeral 34. The portion 3 has a threaded opening 35 to receive a grub screw 36.

With this construction the parts are again assembled together in the manner described with regard to the previous Figures and the releasable locking means are provided by tightening the screw 36 which extends substantially radially to the proximal/distal axis.

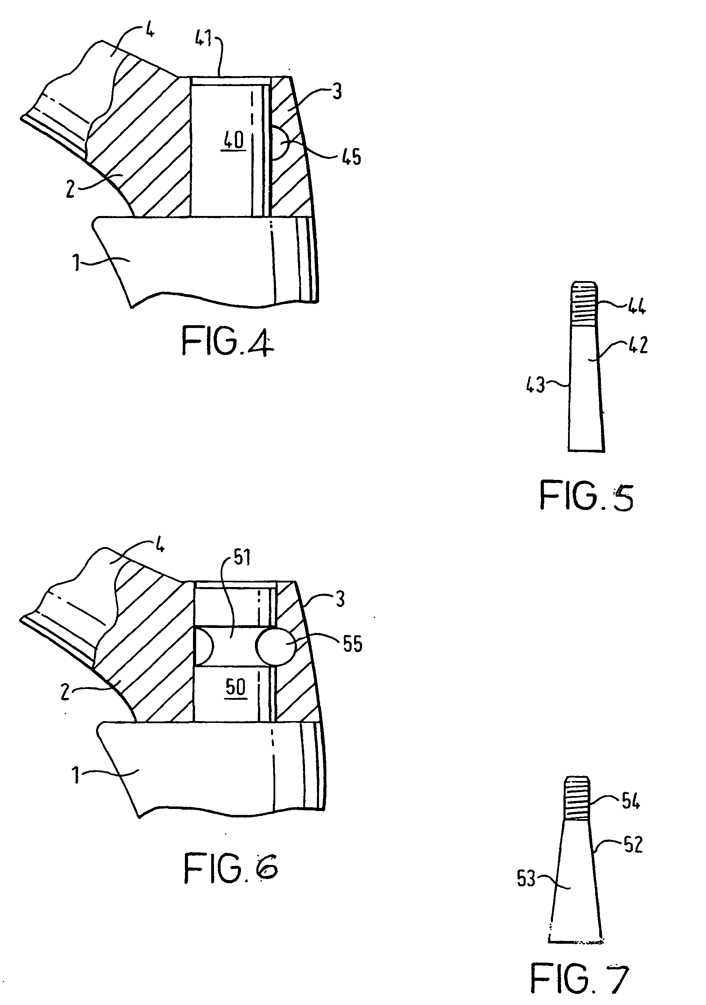

Figures 4 and 5 show another construction according to the invention in which the same reference numerals are again used as those employed in Figure 1 to indicate similar parts but in this construction the tapered boss of Figures 1 to 3 is replaced by a cylindrical boss 40 which extends through an open socket 41 in the proximal component. A cotter pin 42 is again employed, this having a flat side 43 and a threaded end 44. The portion 3 is provided with a suitably shaped opening 45 to receive the pin which is inserted and locking in position in a similar manner to that described with regard to Figure 2.

Figures 6 and 7 also show a construction in which a cotter pin is used but in this arrangement the cylindrical boss 50 is provided with a peripheral groove 51 into which a tapered cotter pin 52 can extend. The cotter pin has a cylindrical tapered shank 53 and is provided with a threaded end 54. The pin is passed through an opening 55 in the portion 3, one side of the pin engaging the opening 55 and the other engaging in the groove 51. Once again it is locked in position by means of a screw on the thread 54.

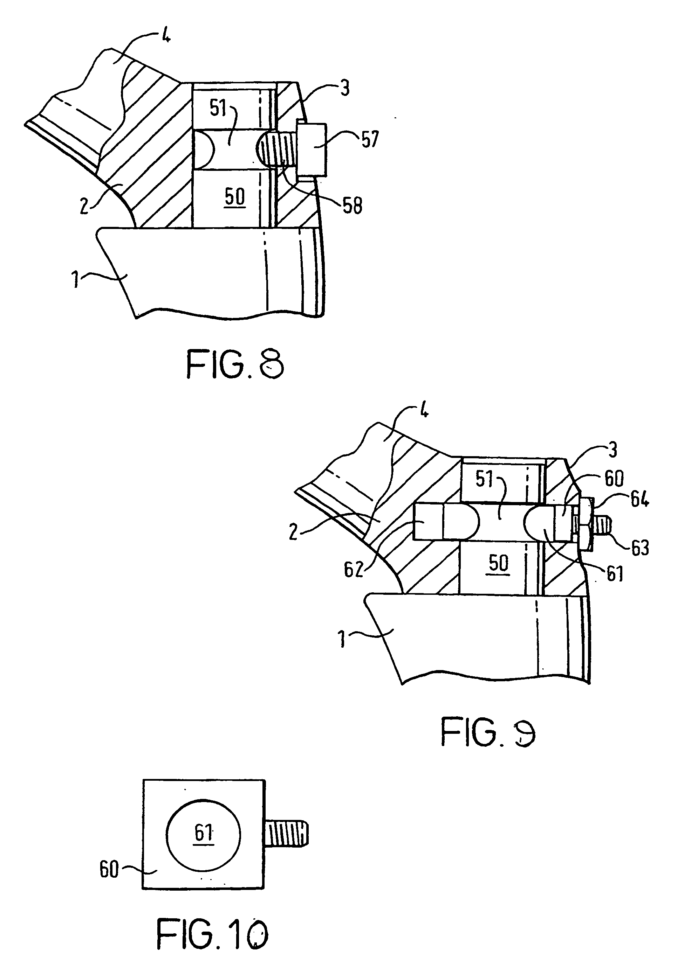

Figure 8 shows another construction using a boss with a groove 51 similar to that shown in Figure 7 but in this construction the cotter pin is replaced by a locking screw 57 which engages the groove by passing through a threaded opening 58 in the portion 3.

Figures 9 and 10 show another construction in which a boss 50 is provided with a groove 51 but the screw 57 of Figure 9 is replaced by a locking plate 60. This plate 60 has a central opening 61 and is located in a slot 62 in the portion 3. The plate is provided with a projecting screw 63 on which is located a tightening nut 64. It will be seen from Figure 1 that the inner edges of the opening 61 are bevelled to accurately locate in the groove 51.

In order to operate this arrangement the plate is located in position in the slot 62 before the proximal component is placed on the boss 50, the angular position is carefully located and the boss is held in position by tightening up the nut 64 which pulls the plate into a position where it can lock against the groove 51.

It will be appreciated that in all the constructions described above it is possible to lock the two parts together without creating a torsional twisting movement to the stem 1.

## Claims

1. A femoral component of a replacement hip joint having a stem (1) for fixing in cement in a medullary cavity and having a separate proximal component (2) provided with a neck (4) for a ball head, and including attachment means for securing the proximal component to the stem which allows selection of an infinite number of angularly displaced positions about a proximal/distal axis, and adapted to allow fixation of the two parts after insertion of the stem into the prepared medullary cavity and without causing torsional loads on the stem, **characterised in that** said attachment means include a boss (20) provided on a proximal portion of said stem (1), a cavity (22) in said proximal component (2) to receive and co-operate with said boss (20) and releasable locking means (25) extending into the cavity (22) and which engage a side of the boss (20) in a direction substantially normal to the longitudinal axis thereof to prevent rotatable axial movement after initial fixing.

2. A femoral component as claimed in claim 1 **characterised in that** said releasable locking means comprises a cotter pin adapted to locate on a boss extension and which extends tangentially to said extension.

3. A femoral component as claimed in claim 1 charactierised in that the releasable locking means is provided by a circumferential groove in said boss which can be engaged by a radially extending lock pin or screw in said proximal component.

4. A femoral component as claimed in any one of claims 1 to 3 **characterised in that** said boss is tapered to accept a tapered cavity on the proximal component

5. A femoral component as claimed in any one of claims 1 to 3 **characterised in that** said boss is cylindrical.

6. A femoral component as claimed in claim 5 **characterised in that** said releasable locking means are provided by a tangentially extending cotter pin located in said proximal component

7. A femoral component as claimed in claim 6 **characterised in that** said cotter pin locates in a groove in said boss.

8. A femoral component as claimed in claim 7 **characterised in that** said releasable locking means comprises a radially extending pin or screw in said proximal component which can engage a peripheral groove in said boss.

9. A femoral component as claimed in claim 5 **characterised in that** said boss is provided with a peripheral groove and the releasable means comprises a locking plate with means to move it radially to engage said groove.

## Patentansprüche

1. Femorale Komponente eines Ersatzhüftgelenks mit einem Schaft (1) zum Befestigen in Zement in einem Markhohlraum und mit einer separaten proximalen Komponente (2), die mit einem Hals (4) für einen Kugelkopf versehen ist, und einschließlich Befestigungsmittel zum Befestigen der proximalen Komponente am Schaft, was die Auswahl einer unendlichen Anzahl von in einem Winkel versetzten Positionen um eine proximale/distale Achse ermöglicht, und dazu angepasst ist, eine Befestigung der zwei Teile nach dem Einsetzen des Schafts in den vorbereiteten Markhohlraum und ohne Verursachen von Torsionsbelastungen am Schaft zu ermöglichen, **dadurch gekennzeichnet, dass** die Befestigungsmittel eine Nabe (20), die an einem proximalen Teil des Schafts (1) bereitgestellt ist, einen Hohlraum (22) in der proximalen Komponente (2), um die Nabe (20) aufzunehmen und mit dieser zusammenzuwirken, und ein lösbares Verriegelungsmittel (25), das sich in den Hohlraum (22) erstreckt und das mit einer Seite der Nabe (20) in einer Richtung im Wesentlichen senkrecht zu deren Längsachse in Eingriff kommt, um eine axiale Drehbewegung nach der anfänglichen Befestigung zu verhindern, einschließen.

2. Femorale Komponente nach Anspruch 1, **dadurch gekennzeichnet, dass** das lösbare Verriegelungsmittel einen Splintbolzen umfasst, der dazu angepasst ist, sich an einer Nabenverlängerung zu befinden, und der sich tangential zur Verlängerung erstreckt.

3. Femorale Komponente nach Anspruch 1, **dadurch gekennzeichnet, dass** das lösbare Verriegelungsmittel durch eine Umfangsnut in der Nabe bereitgestellt wird, die mit einem sich radial erstreckenden Verriegelungsbolzen oder einer Verriegelungsschraube in der proximalen Komponente in Eingriff gebracht werden kann.

4. Femorale Komponente nach einem der Ansprüche 1 bis 3, **dadurch gekennzeichnet, dass** die Nabe verjüngt ist, um einen verjüngten Hohlraum an der proximalen Komponente aufzunehmen.

5. Femorale Komponente nach einem der Ansprüche 1 bis 3, **dadurch gekennzeichnet, dass** die Nabe zylindrisch ist.

6. Femorale Komponente nach Anspruch 5, **dadurch gekennzeichnet, dass** das lösbare Verriegelungsmittel durch einen sich tangential erstreckenden Splintbolzen bereitgestellt wird, der sich in der proximalen Komponente befindet.

7. Femorale Komponente nach Anspruch 6, **dadurch gekennzeichnet, dass** sich der Splintbolzen in einer Nut in der Nabe befindet.

8. Femorale Komponente nach Anspruch 7, **dadurch gekennzeichnet, dass** das lösbare Verriegelungsmittel einen sich radial erstreckenden Bolzen oder eine sich radial erstreckende Schraube in der proximalen Komponente umfasst, der/die mit einer peripheren Nut in der Nabe in Eingriff kommen kann.

9. Femorale Komponente nach Anspruch 5, **dadurch gekennzeichnet, dass** die Nabe mit einer peripheren Nut versehen ist und das lösbare Mittel eine Verriegelungsplatte mit einem Mittel, um sie radial zu bewegen, um mit der Nut in Eingriff zu kommen, umfasst.

## Revendications

1. Composant fémoral pour une articulation de remplacement de hanche comportant une tige (1) à fixer dans du ciment dans une cavité médullaire et possédant un composant proximal séparé (2) pourvu d'un col (4) pour une tête à rotule, et incluant des moyens d'attache pour fixer le composant proximal à la tige permettant la sélection d'un nombre infini de positions angulairement déplacées autour d'un axe proximal/distal, et apte à permettre la fixation des deux parties après insertion de la tige dans la cavité médullaire préparée et sans provoquer de contraintes de torsion sur la tige, **caractérisé en ce que** lesdits moyens d'attache comportent une saillie (20) réalisée sur une partie proximale de ladite tige (1), une cavité (22) dans ledit composant proximal (2) pour recevoir et coopérer avec ladite saillie (20) et un moyen de verrouillage libérable (25) s'étendant dans la cavité (22) et en engagement avec un côté de la saillie dans une direction sensiblement normale à son axe longitudinal pour empêcher un mouvement axial à rotation après fixation initiale.

2. Composant fémoral selon la revendication 1, **caractérisé en ce que** ledit moyen de verrouillage libérable comprend une goupille apte à être disposée sur un prolongement de la saillie et qui s'étend tangentiellement par rapport audit prolongement.

3. Composant fémoral selon la revendication 1, **caractérisé en ce que** ledit moyen de verrouillage libérable comporte une gorge périphérique dans ladite saillie dans laquelle peut s'engager une tige ou une vis de verrouillage s'étendant radialement dans ledit composant proximal.

4. Composant fémoral selon l'une quelconque des revendications 1 à 3, **caractérisé en ce que** ladite saillie est conique pour s'adapter à une cavité conique dans ledit composant proximal.

5. Composant fémoral selon l'une quelconque des revendications 1 à 3, **caractérisé en ce que** ladite saillie est cylindrique.

6. Composant fémoral selon la revendication 5, **caractérisé en ce que** ledit moyen de verrouillage libérable est constitué par une goupille s'étendant tangentiellement dans ledit composant proximal.

7. Composant fémoral selon la revendication 6, **caractérisé en ce que** ladite goupille se place dans une gorge dans ladite saillie.

8. Composant fémoral selon la revendication 7, **caractérisé en ce que** ledit moyen de verrouillage libérable comprend une tige ou vis de verrouillage s'étendant radialement dans ledit composant proximal qui peut s'engager dans une gorge périphérique dans ladite saillie.

9. Composant fémoral selon la revendication 5, **caractérisé en ce que** ladite saillie est pourvue d'une gorge périphérique et le moyen libérable comprend une plaque de verrouillage avec des moyens pour la déplacer radialement pour un engagement dans ladite gorge.
